# FASCICULE DE BREVET EUROPEEN

(11) **EP 3 250 905 B1**
(45) Date de publication et mention de la délivrance du brevet: **15.03.2023**
(21) Numéro de dépôt: 16701636.9
(22) Date de dépôt: 26.01.2016
(51) Int. Cl.: G01N 21/3504, G01N 21/33

(54) **DISPOSITIF TRANSPORTABLE DE MESURE EN LIGNE DE LA CONCENTRATION EN SULFURE D'HYDROGENE D'UN EFFLUENT GAZEUX**
TRAGBARE VORRICHTUNG ZUR INLINE-MESSUNG DER SCHWEFELWASSERSTOFFKONZENTRATION VON ABGAS
PORTABLE APPARATUS FOR ONLINE MEASUREMENT OF THE HYDROGEN SULPHIDE CONCENTRATION OF AN EFFLUENT GAS

(30) Priorité: 27.01.2015 FR 1550615
(43) Date de publication de la demande: 06.12.2017
(73) Titulaire: Arkema France, 92700 Colombes (FR)
(72) Inventeur: HUMBLOT, Francis, 64300 Lanneplaa (FR); SCHMITT, Paul Guillaume, 64230 Lescar (FR); DUBOS, Jean-Luc, 64300 Biron (FR)
(74) Mandataire: Arkema Patent
(86) Numéro de dépôt international: PCT/EP2016/051586
(87) Numéro de publication internationale: WO 2016/120277

(56) Documents cités:
- WO-A1-2014/144038
- CN-A- 101 782 514
- US-B2- 8 071 068
- WELDON V ET AL: "H2S and CO2 gas sensing using DFB laser diodes emitting at 1.57 mum", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH, vol. 29, no. 1, 1 octobre 1995 (1995-10-01), pages 101-107, XP004000858, ISSN: 0925-4005, DOI: 10.1016/0925-4005(95)01669-4
- MODUGNO G ET AL: "Detection of H2S at the ppm level using a telecommunication diode laser", OPTICS COMMUNICATIONS, NORTH-HOLLAND PUBLISHING CO. AMSTERDAM, NL, vol. 145, no. 1-6, 1 janvier 1998 (1998-01-01), pages 76-80, XP004102684, ISSN: 0030-4018, DOI: 10.1016/S0030-4018(97)00461-6
- CHEN W ET AL: "H2S trace concentration measurements using off-axis integrated cavity output spectroscopy in the near-infrared", APPLIED PHYSICS B ; LASERS AND OPTICS, SPRINGER, BERLIN, DE, vol. 90, no. 2, 7 décembre 2007 (2007-12-07), pages 311-315, XP019588237, ISSN: 1432-0649
- JANE HODGKINSON ET AL: "Topical Review;Optical gas sensing: a review;Optical gas sensing: a review", MEASUREMENT SCIENCE AND TECHNOLOGY, IOP, BRISTOL, GB, vol. 24, no. 1, 28 novembre 2012 (2012-11-28), page 12004, XP020236617, ISSN: 0957-0233, DOI: 10.1088/0957-0233/24/1/012004

## Description

### DOMAINE TECHNIQUE

Le domaine technique de l'invention est celui des dispositifs et des méthodes utilisés pour la mesure de la concentration en sulfure d'hydrogène dans un effluent gazeux, en particulier lorsque l'effluent gazeux est issu d'un réacteur utilisé pour hydrotraiter des produits pétroliers.

### ART ANTERIEUR

L'hydrotraitement est un procédé utilisé principalement en raffinage du pétrole et qui a pour but d'enlever des impuretés comme par exemple le soufre contenu dans des coupes pétrolières issues de la distillation du pétrole brut. Une unité d'hydrotraitement comprend un réacteur comprenant deux conduits d'alimentation, l'un destiné à l'introduction de la coupe pétrolière et l'autre destiné à l'introduction d'hydrogène sous pression. Le réacteur contient un catalyseur qui facilite la transformation des composés soufrés en sulfure d'hydrogène H₂S.

Dans un tel procédé, il est nécessaire de sulfurer le catalyseur d'hydrotraitement qui est, le plus souvent, commercialisé sous une forme inactive constitué d'oxydes métalliques de métaux du groupe 6 combinés avec des métaux des groupes 9 et/ou 10, ces oxydes étant supportés sur un solide poreux comme, par exemple une alumine. Cette opération de sulfuration a lieu à chaque changement de catalyseur et à pour but de convertir les oxydes métalliques en sulfures qui constituent les espèces actives dans la réaction d'hydrotraitement. Pour ce faire, il est connu de mettre en contact le catalyseur avec une source de soufre, telle que le diméthyldisulfure (DMDS). Sous l'effet d'une température et d'une pression élevée, le diméthyldisulfure se décompose en sulfure d'hydrogène qui réagit avec le catalyseur pour former les sulfures métalliques souhaités. Une détection de sulfure d'hydrogène formé pendant la sulfuration du catalyseur est nécessaire car elle permet d'avoir une estimation du degré d'avancement de la réaction de sulfuration. D'autre part, il est souhaitable de minimiser la quantité de sulfure d'hydrogène émise lors de la réaction de sulfuration. En outre, la mesure de la concentration en sulfure d'hydrogène est actuellement effectuée par le personnel des raffineries, au mieux toutes les heures, dans des conditions de sécurité qui peuvent être dangereuses, notamment en raison de la toxicité du sulfure d'hydrogène (H₂S). Il a donc été recherché un dispositif et une technique qui permettent une mesure fiable, plus fréquente, dans des conditions accrues de sécurité.

Il existe des dispositifs de mesure de la concentration en ligne de sulfure d'hydrogène dans des effluents gazeux provenant d'unités réalisant l'oxydation du sulfure d'hydrogène en soufre, dites unités de Claus. Ceux-ci sont par exemple décrits dans les documents FR 2 778 743 et FR 2 944 456. Cependant, ces dispositifs sont conçus pour être montés en permanence sur l'installation produisant l'effluent contenant le sulfure d'hydrogène. Ils ne peuvent pas être facilement démontés pour être utilisés rapidement sur une autre unité de Claus.

Le document CN 203595659U décrit un dispositif de mesure de la concentration de sulfure d'hydrogène dans un courant gazeux dont le principe de fonctionnement est fondé sur une analyse spectroscopique laser. Cependant, ce dispositif requiert l'utilisation d'un gaz d'inertage du matériel laser, c'est-à-dire un gaz qui n'est pas comburant avec le gaz à analyser et qui rend le dispositif de mesure anti-explosif. Cependant, l'utilisation d'un gaz d'inertage rend le dispositif complexe.

On recherche donc un dispositif de mesure qui puisse être facilement transportable d'un site à un autre et qui ne nécessite pas l'utilisation d'un gaz d'inertage.

Le document US 8,163,242 décrit un dispositif de mesure de la concentration d'espèces chimiques contenues dans des gaz issus de la décomposition de déchets présents dans les décharges. Cependant, ce document ne donne aucune information sur la technique utilisée pour mesurer spécifiquement la concentration en sulfure d'hydrogène. En outre, ce dispositif de mesure semble faire appel à une technique électrochimique, qui n'est pas adaptée aux mesures de concentrations élevées sans faire appel à un gaz de dilution. Le matériel d'analyse de ce document est "un analyseur chimique", qui par principe implique une réaction chimique irréversible, donc un remplacement fréquent des capteurs chimiques.

Le document WO 2014/144038 décrit un dispositif de mesure en temps réel de la concentration en sulfure d'hydrogène dans une unité d'hydrotraitement de produits pétroliers. Ce dispositif est transportable et est connecté de manière temporaire au conduit de sortie de l'unité d'hydrotraitement. De préférence, la mesure est fondée sur la réaction chimique se produisant entre l'acétate de plomb et le sulfure d'hydrogène. De l'acétate de plomb est déposé sur une bande de papier, donnant ainsi une couleur blanche à la bande. Au cours de la réaction chimique, il se forme du sulfure de plomb de couleur noire. Le degré de noirceur de la bande de papier est proportionnel à la quantité de sulfure d'hydrogène ayant traversé le système de mesure. Ce système de mesure présente néanmoins les inconvénients suivants :
- Il nécessite une chambre de diffusion destinée à diluer, par exemple avec de l'azote, le gaz dont on souhaiter mesurer la concentration en sulfure d'hydrogène. Le gaz de dilution peut soit provenir d'un réseau local, soit être amené avec le dispositif d'analyse transportable. Le fait d'utiliser de l'azote issu d'une raffinerie peut entrainer des erreurs d'analyse en raison de la pollution issue des procédés mis en oeuvre dans la raffinerie.
- Le remplacement du papier usagé par du papier neuf imprégné par de l'acétate de plomb est effectué par un opérateur. Or, l'acétate de plomb fait partie des substances chimiques cancérogènes et/ou mutagènes et/ou toxiques pour la reproduction (substances dites "CMR"). Chaque changement du papier expose l'opérateur à un contact avec l'acétate de plomb, ce qui présente un risque sa santé.
- Ce dispositif ne permet pas de maintenir une bonne précision de mesure de la concentration en H₂S dans le temps, sur une large gamme de concentration allant de 0 à 30 000 ppm.

Ce document indique également que la détection du sulfure d'hydrogène peut être effectuée par une méthode électrochimique.

Le document CN 101782514 divulgue un dispositif de mesure de la concentration en sulfure d'hydrogène dans un gaz naturel, avant et après désulfuration. Ce dispositif comprenant une partie installée de manière fixe sur une installation dans laquelle le gaz contenant H₂S circule, et une partie amovible pouvant être connectée à cette partie fixe. La partie fixe comprend :
- une chambre de mesure dans laquelle s'effectue une mesure de l'absorption par le gaz d'un rayonnement laser;
- une jauge de pression et une vanne de détente permettant de réguler la pression du gaz à analyser à la pression de travail de la chambre de mesure.
La partie amovible comprend :
- un dispositif de production du rayonnement laser et
- un dispositif traitant le signal optique issu de la chambre de mesure.
Cette partie est amovible car elle peut se connecter à la partie fixe à l'aide de deux fibres optiques. Par conséquent, dans ce document, seule la partie dédiée à la production du rayonnement laser et au traitement du signal est amovible.

On recherche donc depuis longtemps dans l'état de l'art à disposer d'un dispositif transportable de mesure en continu précise de la concentration en sulfure d'hydrogène d'un effluent gazeux, généralement comprise entre 100 ppm et 50 000 ppm volumique, de préférence entre 100 ppm et 30 000 ppm, qui puisse être connecté de manière temporaire sur un conduit de transport de cet effluent gazeux et qui n'utilise pas de gaz de dilution. De préférence, ce dispositif ne doit pas nécessiter l'utilisation de substances chimiques dangereuses telles que, entre autre, les "CMR" (Cancérigènes Mutagènes Reprotoxiques). De préférence encore, ce dispositif ne doit pas requérir une alimentation en un gaz d'inertage.

### RESUME DE L'INVENTION

L'invention a pour objet un kit pour la mesure de la concentration en sulfure d'hydrogène d'un gaz susceptible d'en contenir, ledit kit comprenant des modules distincts et connectables les uns aux autres, lesdits modules étant les suivants:
- un module de mesure A comprenant une chambre de mesure M dans laquelle s'effectue la mesure de l'absorption par le gaz d'un rayonnement électromagnétique monochromatique;
- un module de détente B permettant d'amener la pression du gaz à analyser à la pression de travail du module de mesure ;
- un module de régulation de pression C apte à maintenir la pression du gaz dans la chambre de mesure à une valeur située dans la gamme de valeurs de pressions de travail du module de mesure;
- un module d'exploitation D de la mesure de l'absorption, permettant d'obtenir la concentration de sulfure d'hydrogène dans le gaz, ainsi que
- des moyens permettant de connecter les modules les uns aux autres.
Le gaz dont on veut mesurer la concentration en sulfure d'hydrogène est généralement inflammable.

Selon un mode de réalisation, le rayonnement électromagnétique est un rayonnement infrarouge à longueur d'onde fixe émise par un laser, de préférence à une longueur d'onde comprise entre 780 nm et 3000 nm.

Selon un mode de réalisation, le rayonnement électromagnétique est un rayonnement monochromatique situé dans le domaine des longueurs d'onde de l'ultraviolet ou du visible, de préférence dans le domaine des longueurs d'onde comprises entre 100 nm et 380 nm, ou entre 380 et 780 nm, respectivement.

Selon un mode de réalisation,
- le module de détente B possède un conduit d'entrée (2) recevant le gaz à analyser et un conduit de sortie (3) connecté à un conduit d'entrée (4) du module de mesure A ;
- le module de mesure A est connecté électriquement (9) au module d'exploitation D;
- le module de régulation C est connecté mécaniquement au module de mesure A (5, 7) et à un conduit d'évacuation (8) du gaz vers l'extérieur du kit.

Selon un mode de réalisation, le module de détente B permet d'amener la pression du gaz à analyser à la pression de travail du module de mesure comprise entre 500 hPa (0,5 bar) et 2000 hPa (2 bar) relatifs.

Selon un mode de réalisation,
- le module de mesure A a une masse inférieure ou égale à 60 kg, de préférence inférieure ou égale à 55 kg, de préférence encore inférieure ou égale à 50 kg ;
- le module de détente B a une masse inférieure ou égale à 20 kg, de préférence inférieure ou égale à 15 kg, de préférence encore inférieure ou égale à 10 kg ;
- le module de régulation C a une masse inférieure ou égale à 20 kg, de préférence inférieure ou égale à 15 kg, de préférence encore inférieure ou égale à 10 kg ;
- le module d'exploitation D a une masse inférieure ou égale à 50 kg, de préférence inférieure ou égale à 40 kg, de préférence encore inférieure ou égale à 35 kg ;

Le kit peut être utilisé pour la mesure de la concentration en sulfure d'hydrogène d'un gaz susceptible d'en contenir, ledit gaz comprenant au moins 50% d'hydrogène en volume. Le gaz susceptible de contenir du sulfure d'hydrogène peut être un effluent gazeux d'un réacteur utilisé pour la purification d'hydrocarbures issus de procédés du raffinage ou issus de la pétrochimie.

L'invention a également pour objet une méthode de mesure en continu de la concentration en sulfure d'hydrogène d'un effluent gazeux au moyen d'un dispositif amovible apte à être connecté de manière temporaire à une installation produisant ledit effluent gazeux, la méthode comprenant une étape de mesure de l'absorption par l'effluent gazeux d'un rayonnement électromagnétique monochromatique. La méthode de mesure peut utiliser le kit tel que décrit ci-avant, la mesure de la concentration en sulfure d'hydrogène étant réalisée après montage du kit sur l'installation. En fin d'opération, le dispositif amovible peut être facilement démonté de l'installation et ramené sous la forme d'un kit pour être ensuite remonté sur une autre installation sur laquelle le même type de mesure doit être effectué.

Selon un mode de réalisation, le rayonnement électromagnétique est un rayonnement infrarouge à longueur d'onde fixe émise par un laser, de préférence à une longueur d'onde comprise entre 780 nm et 3000 nm.

Selon un mode de réalisation, le rayonnement électromagnétique est un rayonnement monochromatique situé dans le domaine des longueurs d'onde de l'ultraviolet ou du visible, de préférence dans le domaine des longueurs d'onde comprises entre 100 nm et 380 nm, ou entre 380 et 780 nm, respectivement.

Selon un mode de réalisation, la méthode de mesure en continu de la concentration en sulfure d'hydrogène d'un gaz susceptible comprend la mise en oeuvre, à titre de dispositif, du kit selon l'invention.

Selon un mode de réalisation, le gaz est un effluent gazeux issu d'un réacteur utilisé pour la purification avec de l'hydrogène d'hydrocarbures issus de procédés de raffinage ou issus de la pétrochimie.

Selon un mode de réalisation, la méthode est utilisée pour suivre l'avancement et/ou s'assurer de la fin de l'étape de sulfuration d'un catalyseur d'hydrotraitement.

Enfin, l'invention a pour objet une installation dans laquelle un flux gazeux contenant du sulfure d'hydrogène est susceptible d'être généré, caractérisée en ce qu'elle intègre un dispositif obtenu par le montage du kit selon l'invention.

### DESCRIPTION DE LA FIGURE

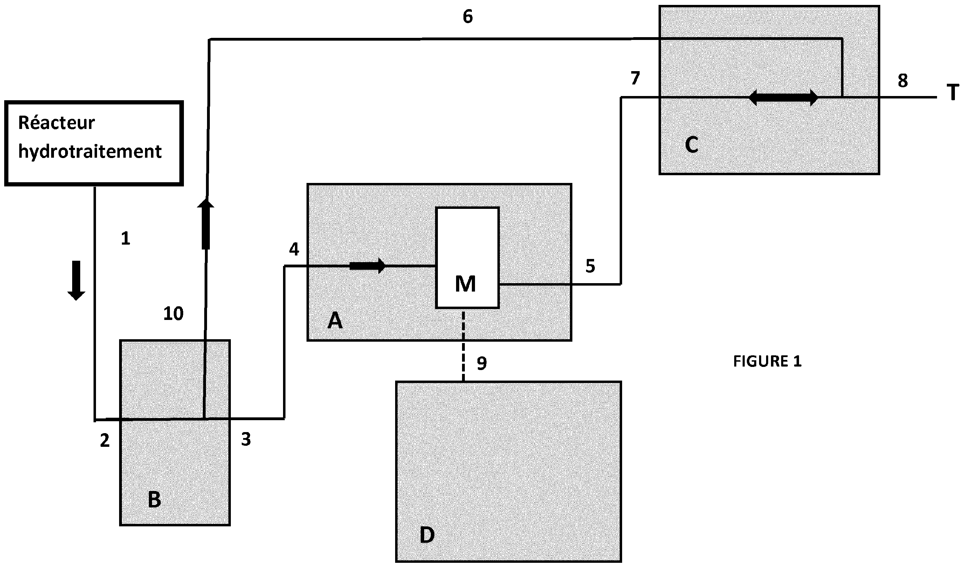

La Figure 1 représente de manière schématique selon un mode de réalisation de l'invention la connexion du dispositif selon l'invention à la sortie d'une unité d'hydrotraitement ainsi que les connexions des différents modules entre eux.

### DESCRIPTION DE MODES DE REALISATION DE L'INVENTION

Le dispositif selon l'invention se présente sous la forme d'un kit comprenant des modules distincts et connectables les uns aux autres, lesdits modules étant les suivants:
- un module de mesure A comprenant une chambre de mesure M dans laquelle s'effectue la mesure de l'absorption par le gaz d'un rayonnement électromagnétique ;
- un module de détente B permettant d'amener la pression du gaz à analyser à la pression de travail du module de mesure ;
- un module de régulation de pression C apte à maintenir la pression du gaz dans la chambre de mesure à une valeur située dans la gamme de valeurs de pressions de travail du module de mesure;
- un module d'exploitation D de la mesure de l'absorption, permettant d'obtenir la concentration de sulfure d'hydrogène dans le gaz, et
- des moyens permettant de connecter les modules les uns aux autres.

La description de l'agencement des différents modules du kit selon un mode de réalisation de l'invention est faite en référence à la Figure 1.

Le module de mesure A comprend un conduit d'entrée (4) apte à être connecté mécaniquement au conduit de sortie (3) du module de détente B et un conduit de sortie (5) apte à être connecté mécaniquement à un conduit (7) du module de régulation C. La mesure de la concentration en sulfure d'hydrogène est effectuée dans une chambre de mesure M selon le principe connu de la spectrophotométrie. Selon ce principe, une substance est traversée par un rayonnement électromagnétique et on mesure l'absorption de ce rayonnement électromagnétique par la substance. Cette chambre de mesure est constituée d'une capacité en acier inoxydable, généralement de forme tubulaire et d'une longueur comprise entre 5 et 80 cm, de préférence entre 10 et 50 cm. Cette chambre de mesure comprend une source émettrice de rayonnement électromagnétique et un capteur de ce rayonnement convertissant le rayonnement en un signal électrique. La diode émettrice et le capteur peuvent être fixés sur les parois de la cellule de mesure l'un en face de l'autre ou côte-à-côte. Dans ce dernier cas, le rayonnement électromagnétique se reflète sur un miroir qui renvoie le rayonnement vers le capteur. Cette configuration augmente le trajet optique et la sensibilité de la mesure. Le choix de l'une ou l'autre des configurations dépend de la longueur d'onde de la radiation électromagnétique et de son coefficient d'absorption du rayonnement par le sulfure d'hydrogène, ainsi que de la gamme de mesure de la concentration de sulfure d'hydrogène choisie. Optionnellement, la source de radiation électromagnétique et le capteur peuvent être déportés de la chambre de mesure en ajoutant deux fibres optiques pour amener la radiation électromagnétique de la source à la chambre de mesure et ramener ce même rayonnement, après absorption, vers le capteur.

La radiation électromagnétique peut être :
- soit une lumière laser émettant dans l'infra-rouge à une longueur d'onde fixe comprise entre 780 et 3000 nm ;
- soit une radiation électromagnétique monochromatique émettant dans le domaine ultraviolet ou le domaine visible, c'est-à-dire dans le domaine des longueurs d'onde comprises entre 100 nm et 380 nm, ou entre 380 et 780 nm, respectivement.

On peut utiliser un analyseur laser infra-rouge : Modèle "SS2100 TDL Gas Analyser" commercialisé par la Société Spectra Sensor, ou un analyseur UV-Visible Modèle "OMA-300 Hydrogen Sulfide Analyzer" commercialisé par la Société Applied Analytics.

Le module A a généralement une masse inférieure ou égale à 60 kg, de préférence inférieure ou égale à 55 kg, de préférence encore inférieure ou égale à 50 kg.

Le module de détente B comporte :
- un conduit d'entrée (2) apte à être connecté mécaniquement au conduit (1) lui-même monté en dérivation du conduit de sortie des gaz issus d'un réacteur d'hydrotraitement.
- un conduit de sortie (3) apte à être connecté mécaniquement au conduit d'entrée (4) du module de mesure A. Il a pour fonction de faire diminuer la pression du gaz à la pression de travail du module de mesure qui est généralement comprise entre 500 hPa et 2000 hPa.
- éventuellement un conduit de sortie (10) utilisé pour évacuer une surpression non compatible avec la pression de travail du module de mesure qui est généralement comprise entre 500 hPa et 2000 hPa. Il joue le rôle d'organe de sécurité.

Le module de détente B a généralement une masse inférieure ou égale à 20 kg, de préférence inférieure ou égale à 15 kg, de préférence encore inférieure ou égale à 10 kg

Le module de régulation C possède un conduit d'entrée (6) apte à être optionnellement relié mécaniquement au conduit (10) et deux conduits (7,8). L'un des deux conduits (8) est un conduit d'évacuation du gaz ou servant à amener le gaz vers un dispositif de destruction du gaz, telle qu'une torche assurant la combustion du gaz. L'autre conduit (7) est apte à recevoir le gaz issu du le module de mesure A en gaz à analyser. Le module de régulation permet de réguler la pression dans la chambre de mesure du module A à une valeur située dans la gamme de pressions de travail du module de mesure. Si la pression est inférieure à la limite inférieure de la gamme de pressions de travail, le module de détente B envoie du gaz dans la chambre de mesure par l'intermédiaire du conduit (3). Si celle-ci dépasse la limite supérieure de la gamme de pressions de travail, du gaz est injecté dans le conduit (10) puis (8) d'évacuation ou de destruction du gaz. Le conduit (8) reçoit également le gaz provenant du module de mesure A lorsque ce dernier fonctionne dans sa gamme de pression de travail.

Le module de régulation C a généralement une masse inférieure ou égale à 20 kg, de préférence inférieure ou égale à 15 kg, de préférence encore inférieure ou égale à 10 kg.

Le module D d'exploitation du signal spectroscopique est connecté électriquement au module B par l'intermédiaire de la liaison électrique (9). Il convertit la mesure de l'absorption provenant du module A en une concentration en sulfure d'hydrogène. Dans un mode de réalisation préféré de l'invention, la teneur instantanée en sulfure d'hydrogène peut être indiquée par écran, avantageusement intégré au module D.

Le module d'exploitation D a généralement une masse inférieure ou égale à 50 kg, de préférence inférieure ou égale à 40 kg, de préférence encore inférieure ou égale à 35 kg.

Les résultats d'analyse générés par le module D d'exploitation peuvent être transférés pendant ou après analyse, en totalité ou en partie, vers un ordinateur au moyen d'une transmission sans fil par exemple de type Wifi, BlueTooth, et autre, ou d'une transmission filaire au moyen d'une carte mémoire.

Les modules A et D peuvent être chacun contenus dans un boîtier métallique épais antidéflagrant au sens de la norme 94/9/EC, c'est-à-dire qu'une éventuelle explosion reste confinée à l'intérieur du boîtier qui n'est pas endommagé.

L'effluent gazeux contenant le sulfure d'hydrogène à analyser peut être issu d'un réacteur d'hydrotraitement HDT. L'effluent gazeux contient généralement entre 100 et 50 000 ppm volumique de sulfure d'hydrogène, de préférence entre 100 et 30 000 ppm volumique, de préférence encore entre 1 000 et 20 000 ppm volumique.

À la sortie du réacteur d'hydrotraitement, cet effluent gazeux riche en hydrogène est séparé des hydrocarbures liquides dans une capacité dans laquelle les composés liquides s'accumulent en pied alors que les gaz non condensables sont évacués et tête. Ces gaz sont, en général, recomprimés par un compresseur pour être réinjectés en amont du ou des réacteurs d'hydrotraitement. Ces gaz constituent l'effluent gazeux dont on cherche à connaître la teneur en sulfure d'hydrogène. Pour cela, on place en dérivation du flux gazeux principal, un circuit terminé par un conduit de connexion (1). Ce conduit (1) est connecté au conduit (2) d'entrée du module B. Le gaz à analyser est donc acheminé dans le module de détente B par le conduit d'entrée (2) puis est transféré par le conduit de sortie (3) dans le module de mesure A comprenant la chambre de mesure M. Il est ensuite envoyé dans le module de régulation C. Il est évacué du module de régulation C et est envoyé vers la torche (T) pour y être brûlé. Le module de régulation C maintient la pression du gaz dans le module de mesure à une valeur constante en ajustant la quantité de gaz envoyée dans la chambre de mesure M et celle envoyée vers la torche T. Dans le cas où le gaz à analyser serait dans le conduit (1) à une pression compatible avec la pression de travail du module de mesure A, le module de détente serait optionnel.

Les connexions entre les modules A, B et C ainsi que celles pour se connecter au point de prélèvement du gaz (1) ainsi qu'au circuit d'évacuation par la torche T sont assurées par des tuyaux flexibles pouvant supporter une pression maximum de 25 MPa. Ils sont équipés de raccords rapides et d'obturateurs prévus pour être connectés et déconnectés fréquemment.

Le module D est connecté au module A par des câbles électriques.

Selon un mode de réalisation, les modules A et D sont solidaires dans un même boîtier auquel sont connectés les modules B et C.

Selon un mode de réalisation, les modules A et C sont solidaires dans un même boîtier auquel sont connectés les modules B et D.

Selon un mode de réalisation, les modules A, C et D sont solidaires dans un même boîtier auquel est connecté le module B.

Le dispositif selon l'invention peut être facilement connecté sans ouverture de circuit, ce qui limite le risque d'exposition d'un opérateur à des gaz dangereux.

Dans le cadre de la sulfuration du catalyseur d'hydrotraitement, on réalise l'injection de DMDS au débit demandé par la raffinerie pendant toute la procédure d'activation du catalyseur, qui dure généralement de 1 à 2 jours, et simultanément avec l'injection de DMDS, on utilise le dispositif selon l'invention pour mesurer et suivre l'augmentation de la concentration de sulfure d'hydrogène au cours du temps. Lorsque la concentration de sulfure d'hydrogène comparée à la quantité de DMDS injectée, montre qu'il n'y a plus de soufre fixé par le catalyseur, cela signifie qu'il n'est plus utile de continuer à injecter du DMDS.

Le dispositif selon l'invention possède les avantages suivants :
a) Il peut être déconnecté de l'installation produisant l'effluent gazeux contenant le sulfure d'hydrogène après que la mesure de la concentration ait été effectuée et il peut être transporté rapidement sur un autre site pour y être utilisé. Le dispositif selon l'invention se caractérise par le fait qu'il est aisément transportable par camion, avion, voiture ou bateau, en raison de son poids limité (par exemple 2 modules de moins de 50 kg chacun et 2 modules de moins de 10 kg chacun) et par ses dimensions réduites, c'est-à-dire généralement inférieures à 800 mm x 600 mm x 400 mm. Il peut aussi être transporté par un homme, sans que celui-ci n'ait à un recourir à un dispositif de manutention.
b) Il est "autonome" en ce qu'il ne nécessite l'emploi d'aucun gaz, autre que celui faisant l'objet de l'analyse. En particulier :
   - Il ne nécessite aucun gaz vecteur, ce qui représente un avantage par rapport aux techniques de mesure par chromatographie en phase gazeuse qui nécessitent l'emploi d'hydrogène ou d'hélium.
   - Il ne nécessite pas obligatoirement l'utilisation d'un gaz d'inertage pour le rendre conforme à la règlementation ATEX (ATmosphères EXplosives) relative aux appareils destinés à être utilisés en atmosphères explosives.
   - Il ne nécessite pas l'utilisation d'un gaz de dilution, contrairement aux techniques de mesure par électrochimie ou par réaction entre le sulfure d'hydrogène et l'acétate de plomb, telles que mentionnées dans le document WO 2014/144038.
c) Il se caractérise par une incertitude de mesure faible (<100 ppm) sur toute la gamme de mesure souhaitée pour l'application à l'hydrotraitement, contrairement aux techniques nécessitant un gaz de dilution, telles que celle basée sur l'utilisation d'un papier imprégné d'acétate de plomb.
d) Il permet une analyse en continu de la concentration de sulfure d'hydrogène, avec une fréquence de mesure se situant dans la plage allant de 5 à 30 secondes suivant le débit de gaz à analyser. Grâce à cette mesure en continu, le raffineur peut réagir plus rapidement à des dérives de la teneur en sulfure d'hydrogène pour ajuster, par exemple, le débit d'injection de DMDS. On évite ainsi des concentrations élevées de sulfure d'hydrogène, supérieures à 3% en volume, néfastes pour la section de recompression et des concentrations trop faibles, inférieures à 0,1% qui peuvent endommager le catalyseur lorsque la température du réacteur dépasse 250°C.
e) Il ne requiert pas l'utilisation de composés chimiques dangereux, tels que l'acétate de plomb.
f) Il est conforme à la règlementation ATEX (norme européenne anti-déflagrante ou anti-explosion) tout en étant à la fois facilement transportable et peu encombrant. En effet, on ne trouve chez les fournisseurs de matériel d'analyse industriel que du matériel fixe et encombrant qui réponde au minimum à la norme ATex II 2 G Ex d II B + H2T4. La classification ATEX d'un équipement est basée sur la directive européenne 94/9/EC.

Le kit selon l'invention répond au point "p" (suppression de l'atmosphère explosive) de la classification ATEX et/ou au point "e" (suppression de la source d'inflammation) et/ou au point "d" (antidéflagrant, non propagation de l'inflammation). De préférence, le kit selon l'invention répond au moins au point "d" de la classification ATEX par l'emploi de boitiers antidéflagrants autour des modules A et D.

La description de l'invention a été faite dans ce qui précède en prenant comme exemple la mesure du sulfure d'hydrogène dans un effluent gazeux issu d'une unité d'hydrotraitement de coupes pétrolières. Cependant, l'invention ne se limite pas à cette application et peut être mise en oeuvre dans les procédés de raffinage du pétrole dans lesquels de l'hydrogène est utilisé pour purifier des hydrocarbures. Elle peut aussi être mise en oeuvre pour mesurer la quantité de sulfure d'hydrogène présente dans un effluent gazeux issu d'une unité mettant en oeuvre la réaction d'oxydation catalytique de sulfure d'hydrogène en soufre (réaction de Claus). Elle peut aussi être utilisée en pétrochimie ou dans des procédés de transformation de produits d'origine naturelle ("bioraffinerie"). Elle peut également être mise en oeuvre dans les domaines de l'industrie produisant du sulfure d'hydrogène, tels que le traitement des eaux usées, les hauts fourneaux, la papeterie, la tannerie.

## Revendications

1. Kit transportable pour la mesure de la concentration en sulfure d'hydrogène d'un gaz susceptible d'en contenir, ledit kit comprenant des modules distincts et connectables les uns aux autres, lesdits modules étant les suivants:
- un module de mesure A comprenant une chambre de mesure M dans laquelle s'effectue la mesure de l'absorption par le gaz d'un rayonnement électromagnétique monochromatique;
- un module de détente B permettant d'amener la pression du gaz à analyser à la pression de travail du module de mesure ;
- un module de régulation de pression C apte à maintenir la pression du gaz dans la chambre de mesure à une valeur située dans la gamme de valeurs de pressions de travail du module de mesure;
- un module d'exploitation D de la mesure de l'absorption, permettant d'obtenir la concentration de sulfure d'hydrogène dans le gaz, ainsi que
- des moyens permettant de connecter les modules les uns aux autres,
ledit kit présentant en outre les caractéristiques suivantes :
- le module de détente B possède un conduit d'entrée (2) recevant le gaz à analyser et un conduit de sortie (3) connecté à un conduit d'entrée (4) du module de mesure A ;
- le module de mesure A est connecté électriquement (9) au module d'exploitation D; et
- le module de régulation C est connecté mécaniquement au module de mesure A (5, 7) et à un conduit d'évacuation (8) du gaz vers l'extérieur du kit ;
ledit kit répondant au point "p" (suppression de l'atmosphère explosive) et/ou au point "e" (suppression de la source d'inflammation) et/ou au point "d" (antidéflagrant, non propagation de l'inflammation) de la classification ATEX.

2. Kit selon la revendication 1, dans lequel le rayonnement électromagnétique est un rayonnement infrarouge à longueur d'onde fixe émise par un laser, de préférence à une longueur d'onde comprise entre 780 nm et 3000 nm.

3. Kit selon la revendication 1, dans lequel le rayonnement électromagnétique est un rayonnement monochromatique situé dans le domaine des longueurs d'onde de l'ultraviolet ou du visible, de préférence dans le domaine des longueurs d'onde comprises entre 100 nm et 380 nm, ou entre 380 et 780 nm, respectivement.

4. Kit selon l'une des revendications précédentes, dans lequel le module de détente B permet d'amener la pression du gaz à analyser à la pression de travail du module de mesure comprise entre 500 hPa (0,5 bar) et 2000 hPa (2 bar) relatifs.

5. Kit selon l'une des revendications précédentes, dans lequel:
- le module de mesure A a une masse inférieure ou égale à 60 kg, de préférence inférieure ou égale à 55 kg, de préférence encore inférieure ou égale à 50 kg ;
- le module de détente B a une masse inférieure ou égale à 20 kg, de préférence inférieure ou égale à 15 kg, de préférence encore inférieure ou égale à 10 kg ;
- le module de régulation C a une masse inférieure ou égale à 20 kg, de préférence inférieure ou égale à 15 kg, de préférence encore inférieure ou égale à 10 kg ;
- le module d'exploitation D a une masse inférieure ou égale à 50 kg, de préférence inférieure ou égale à 40 kg, de préférence encore inférieure ou égale à 35 kg ;

6. Méthode de mesure en continu de la concentration en sulfure d'hydrogène d'un effluent gazeux au moyen d'un dispositif amovible apte à être connecté de manière temporaire à une installation produisant ledit effluent gazeux, la méthode comprenant la mise en oeuvre, à titre de dispositif amovible, du kit selon l'une des revendications 1 à 5.

7. Méthode de mesure selon la revendication 6, dans laquelle le gaz est un effluent gazeux issu d'un réacteur utilisé pour la purification avec de l'hydrogène d'hydrocarbures issus de procédés de raffinage ou issus de la pétrochimie.

8. Méthode de mesure selon l'une des revendications 6 ou 7, utilisée pour suivre l'avancement et/ou s'assurer de la fin de l'étape de sulfuration d'un catalyseur d'hydrotraitement.

9. Installation dans laquelle un flux gazeux contenant du sulfure d'hydrogène est susceptible d'être généré, **caractérisée en ce qu'**elle intègre un dispositif obtenu par le montage du kit défini dans l'une des revendications 1 à 5.

10. Utilisation du kit selon l'une des revendications 1 à 5, pour la mesure de la concentration en sulfure d'hydrogène d'un gaz susceptible d'en contenir, ledit gaz comprenant au moins 50% d'hydrogène en volume.

11. Utilisation selon la revendication 10, dans laquelle le gaz susceptible de contenir du sulfure d'hydrogène est un effluent gazeux d'un réacteur utilisé pour la purification d'hydrocarbures issus de procédés du raffinage ou issus de la pétrochimie.

## Patentansprüche

1. Transportierbares Kit zum Messen der Schwefelwasserstoffkonzentration eines Gases, das Schwefelwasserstoff enthalten könnte, wobei das Kit einzelne und miteinander verbindbare Module umfasst, wobei es sich dabei um folgende Module handelt:
- ein Messmodul A, das eine Messkammer M umfasst, in der die Messung der Absorption einer monochromatischen elektromagnetischen Strahlung durch das Gas stattfindet;
- ein Entspannungsmodul B, mit dem sich der Druck des zu analysierenden Gases auf den Arbeitsdruck des Messmoduls bringen lässt;
- ein Druckregelmodul C, mit dem sich der Druck des Gases in der Messkammer auf einem Wert halten lässt, der im Wertebereich des Arbeitsdrucks des Messmoduls liegt;
- ein Modul zur Auswertung D der Absorptionsmessung, mit dem sich die Schwefelwasserstoffkonzentration im Gas erhalten lässt, sowie
- Mittel, mit denen sich die Module miteinander verbinden lassen,
wobei das Kit ferner folgende Eigenschaften aufweist:
- das Entspannungsmodul B besitzt eine Einlassleitung (2), die das zu analysierende Gas aufnimmt, und eine Auslassleitung (3), die mit einer Einlassleitung (4) des Messmoduls A verbunden ist;
- das Messmodul A ist elektrisch (9) mit dem Auswertungsmodul D verbunden; und
- das Regelmodul C ist mechanisch mit dem Messmodul A (5, 7) und mit einer Leitung zum Abführen (8) des Gases aus dem Kit heraus verbunden;
wobei das Kit Punkt "p" (Unterdrückung der explosionsfähigen Atmosphäre) und/oder Punkt "e" (Unterdrückung der Zündquelle) und/oder Punkt "d" (explosionsgeschützt, keine Ausbreitung der Entzündung) nach der ATEX-Kennzeichnung entspricht.

2. Kit nach Anspruch 1, wobei die elektromagnetische Strahlung eine Infrarotstrahlung mit einer festen Wellenlänge ist, die von einem Laser emittiert wird, vorzugsweise mit einer Wellenlänge zwischen 780 nm und 3000 nm.

3. Kit nach Anspruch 1, wobei die elektromagnetische Strahlung eine monochromatische Strahlung ist, die im Wellenlängenbereich der ultravioletten oder der sichtbaren Strahlung liegt, vorzugsweise im Wellenlängenbereich zwischen 100 nm und 380 nm beziehungsweise zwischen 380 und 780 nm.

4. Kit nach einem der vorhergehenden Ansprüche, wobei sich mit dem Entspannungsmodul B der Druck des zu analysierenden Gases auf den Arbeitsdruck des Messmoduls zwischen 500 hPa (0,5 bar) und 2000 hPa (2 bar) Relativdruck bringen lässt.

5. Kit nach einem der vorhergehenden Ansprüche, wobei:
- das Messmodul A eine Masse kleiner gleich 60 kg, vorzugsweise kleiner gleich 55 kg, bevorzugter kleiner gleich 50 kg aufweist;
- das Entspannungsmodul B eine Masse kleiner gleich 20 kg, vorzugsweise kleiner gleich 15 kg, bevorzugter kleiner gleich 10 kg aufweist;
- das Regelmodul C eine Masse kleiner gleich 20 kg, vorzugsweise kleiner gleich 15 kg, bevorzugter kleiner gleich 10 kg aufweist;
- das Auswertungsmodul D eine Masse kleiner gleich 50 kg, vorzugsweise kleiner gleich 40 kg, bevorzugter kleiner gleich 35 kg aufweist.

6. Verfahren zur kontinuierlichen Messung der Schwefelwasserstoffkonzentration eines gasförmigen Austrags mit einer abnehmbaren Vorrichtung, die sich vorübergehend an einer Anlage anschließen lässt, die den gasförmigen Austrag erzeugt, wobei das Verfahren das Einsetzen des Kits nach einem der Ansprüche 1 bis 5 mit der abnehmbaren Vorrichtung umfasst.

7. Messverfahren nach Anspruch 6, wobei das Gas ein gasförmiger Austrag aus einem Reaktor ist, der zur Reinigung, mit Wasserstoff, von Kohlenwasserstoffen aus Raffinerieverfahren oder petrochemischen Verfahren eingesetzt wird.

8. Messverfahren nach einem der Ansprüche 6 oder 7, das zum Überwachen des Fortschreitens und/oder zum Sicherstellen des Abschlusses des Schritts zur Sulfurierung eines Hydrotreating-Katalysators eingesetzt wird.

9. Anlage, in der ein gasförmiger Strom, der Schwefelwasserstoff enthält, erzeugt werden könnte, **dadurch gekennzeichnet, dass** darin eine Vorrichtung integriert ist, die durch Montage des in einem der Ansprüche 1 bis 5 definierten Kits erhalten wird.

10. Verwendung des Kits nach einem der Ansprüche 1 bis 5, zum Messen der Schwefelwasserstoffkonzentration eines Gases, das Schwefelwasserstoff enthalten könnte, wobei das Gas mindestens 50 Volumen-% Wasserstoff enthält.

11. Verwendung nach Anspruch 10, wobei das Gas, das Schwefelwasserstoff enthalten könnte, ein gasförmiger Austrag eines Reaktors ist, der zur Reinigung von Kohlenwasserstoffen aus Raffinerieverfahren oder petrochemischen Verfahren eingesetzt wird.

## Claims

1. Portable kit for measuring the hydrogen sulfide concentration of a gas likely to contain it, said kit comprising separate modules that can be connected to one another, said modules being the following:
- a measurement module A comprising a measurement chamber M wherein the absorption by the gas of a monochromatic electromagnetic radiation is measured;
- an expansion module B that makes it possible to bring the pressure of the gas to be analysed to the working pressure of the measurement module;
- a pressure-regulating module C capable of maintaining the pressure of the gas in the measurement chamber at a value lying within the range of working pressure values of the measurement module;
- a module D for processing the absorption measurement, which makes it possible to obtain the concentration of hydrogen sulfide in the gas, and also
- means which make it possible to connect the modules to one another,
said kit further having the following characteristics:
- the expansion module B has an inlet duct (2) that receives the gas to be analysed and an outlet duct (3) connected to an inlet duct (4) of the measurement module A;
- the measurement module A is electrically connected (9) to the processing module D; and
- the regulating module C is mechanically connected to the measurement module A (5, 7) and to a duct (8) for discharging the gas out of the kit;
said kit satisfying point "p" (suppression of the explosive atmosphere) and/or point "e" (suppression of the source of combustion) and/or point "d" (explosion-proof, no propagation of combustion) of the ATEX classification.

2. Kit according to Claim 1, wherein the electromagnetic radiation is a fixed wavelength infrared radiation emitted by a laser, preferably having a wavelength between 780 nm and 3000 nm.

3. Kit according to Claim 1, wherein the electromagnetic radiation is a monochromatic radiation lying in the ultraviolet or visible wavelength range, preferably in the range of wavelengths between 100 nm and 380 nm, or between 380 and 780 nm, respectively.

4. Kit according to one of the preceding claims, wherein the expansion module B makes it possible to bring the pressure of the gas to be analysed to the working pressure of the measurement module of between 500 hPa (0.5 bar) relative and 2000 hPa (2 bar) relative.

5. Kit according to one of the preceding claims, wherein:
- the measurement module A has a weight of less than or equal to 60 kg, preferably less than or equal to 55 kg, more preferably less than or equal to 50 kg;
- the expansion module B has a weight of less than or equal to 20 kg, preferably less than or equal to 15 kg, more preferably less than or equal to 10 kg;
- the regulating module C has a weight of less than or equal to 20 kg, preferably less than or equal to 15 kg, more preferably less than or equal to 10 kg;
- the processing module D has a weight of less than or equal to 50 kg, preferably less than or equal to 40 kg, more preferably less than or equal to 35 kg.

6. Method for the continuous measurement of the hydrogen sulfide concentration of an off-gas using a detachable device capable of being connected temporarily to a plant producing said off-gas, the method comprising the use, as a detachable device, of the kit according to one of Claims 1 to 5.

7. Measurement method according to Claim 6, wherein the gas is an off-gas from a reactor used for the purification, with hydrogen, of hydrocarbons resulting from refining processes or from petrochemistry.

8. Measurement method according to either of Claims 6 and 7, used for monitoring the progression and/or ensuring the end of the step of sulfiding a hydrotreating catalyst.

9. Plant in which a gas stream containing hydrogen sulfide is likely to be generated, **characterized in that** it integrates a device obtained by the mounting of the kit defined in one of Claims 1 to 5.

10. Use of the kit according to one of Claims 1 to 5, for measuring the hydrogen sulfide concentration of a gas likely to contain it, said gas comprising at least 50% hydrogen by volume.

11. Use according to Claim 10, wherein the gas likely to contain hydrogen sulfide is an off-gas of a reactor used for the purification of hydrocarbons resulting from refining processes or from petrochemistry.
